(19) Europäisches Patentamt / European Patent Office / Office européen des brevets

(11) **EP 1 478 350 B1**

(12) **EUROPEAN PATENT SPECIFICATION**

(45) Date of publication and mention of the grant of the patent:
**18.04.2007 Bulletin 2007/16**

(51) Int Cl.:
***A61K 9/28*** (2006.01)

(86) International application number:
**PCT/GB2003/000855**

(21) Application number: **03706749.3**

(22) Date of filing: **28.02.2003**

(87) International publication number:
**WO 2003/072086 (04.09.2003 Gazette 2003/36)**

(54) **PHARMACEUTICAL DOSAGE FORMS COMPRISING TABLET CORE HAVING A TENSILE STRENGTH BELOW 38 N/SQCM AND A COATING TO PROTECT THE SOFT CORE**

PHARMAZEUTISCHE DARREICHUNGSFORMEN, WELCHE EINEN TABLETTENKERN MIT EINER BRUCHFESTIGKEIT UNTER 38N/QCM UND EINEN ÜBERZUG ZUM SCHUTZ DES WEICHEN KERNS ENTHALTEN

FORMES DE DOSES PHARMACEUTIQUES COMPRENANT UN NOYAU DE TABLETTE DONT LA RESISTANCE A LA RUPTURE EST INFERIEURE A 38 N/SQCM AVEC UN ENROBAGE DE PROTECTION DU NOYAU FRIABLE

(84) Designated Contracting States:
**AT BE BG CH CY CZ DE DK EE ES FI FR GB GR HU IE IT LI LU MC NL PT SE SI SK TR**

(30) Priority: **28.02.2002 GB 0204772**

(43) Date of publication of application:
**24.11.2004 Bulletin 2004/48**

(73) Proprietor: **Phoqus Pharmaceuticals Limited**
**West Malling,**
**Kent ME19 4PQ (GB)**

(72) Inventors:
• **TIAN, Wei**
**Malesbury, SN16 9QS (GB)**
• **LANGRIDGE, John**
**Manchester, M29 7EH (GB)**
• **WHITEMAN, Marshall**
**Ditton, Kent ME20 6QS (GB)**

(74) Representative: **Bowman, Paul Alan**
**Lloyd Wise**
**Commonwealth House,**
**1-19 New Oxford Street**
**London WC1A 1LW (GB)**

(56) References cited:
**WO-A-92/14451**      **WO-A-93/13758**
**WO-A-03/007918**      **US-B1- 6 277 409**

**Description**

This invention relates to pharmaceutical dosage forms and in particular to solid pharmaceutical dosage forms such as tablets with improved structural integrity.

Tablets are generally made by compressing a powder mixture under high pressure in order to form a tablet having the necessary crushing strength for the handling required during packaging and distribution. The powder mixture generally comprises a pharmaceutically active ingredient and one or more pharmaceutically acceptable adjuvants e.g. binder, diluent, disintegrant, lubricant, wetting agent, glidant, surfactant, release aid, colourant etc.

There are certain types of pharmaceutical formulations which could be conveniently administered in the form of a tablet but are not readily susceptible to conventional tableting techniques. For example, rapidly dissolving or disintegrating formulations, which are intended to disintegrate or dissolve within a few seconds, should ideally have a porous, low density structure which is not compatible with high pressure tableting techniques. Similarly, beads or microcapsules of active ingredient could be conveniently administered in the form of a tablet but they are susceptible to damage under the high pressures involved with conventional tableting techniques. When formulations are tableted using reduced pressures there may be a significant reduction in the strength of the resulting tablet which is disadvantageous and may be totally unacceptable. For example, the tablets may disintegrate during subsequent handling, storage, transport and packaging, particularly if they are loose in a container. Also, the tablets may disintegrate upon handling by the patient e.g. when extracting from a blister pack or the like.

US-A-6207199 discloses a process for making a rapidly dissolving dosage form in which a porous particulate powder matrix comprising at least two polymeric components which serve as the dosage form matrix is produced. The polymeric components have different solubilities. A pharmaceutical compound is combined with the powder and other additives may be added and the mixture is formed in to a dosage form e.g. tablet by mild compression. Due to the porous nature of tablet, the tablet tends to be rather fragile and breakable and generally benefits from the added protection afforded by a coating. The coating may comprises a polymer, such as a polyvinyl alcohol or a polyvinylpyrrolidone which, when applied forms a polymeric net over and into the tablets. This net maintains the tablet intact but does not inhibit the capillary uptake of the tablet once placed in an aqueous environment. The polymer is applied to the tablet in solution e.g. by dropping, by spraying or by passing the tablet through an environment saturated with the coating agent. Alternatively, the tablet may be formed by a sintering process in which one or more polyethylene glycols is mixed with the drug, support matrix mixture. After forming the tablet, the tablet is heated briefly e.g. at 90°C for ten minutes. The polyethylene glycol within the mixture melts forming a thin coating on the tablet.

WO01/10418 discloses a rapidly disintegratable tablet comprising at least one active substance and a mixture of excipients which include at least one binding polymer, the tablet is sintered for a sufficient time and temperature to allow the binding polymer to change status or melt and allow the polymer to resolidify as the temperature is reduced to ambient temperature thereby providing excellent tablet binding characteristics. The preferred binding polymer is polyethylene glycol.

Membrane coated beads or microcapsules are often incorporated into hard capsules to provide immediate or controlled release dosage forms. Tablets containing these beads or microcapsules have several advantages over capsules for the speed and cost of manufacturing, and also the ability to incorporate a high amount of active ingredients. Furthermore, the beads containing tablets do not rely on the use of gelatine, which is objectionable to certain patient groups, However, compaction of beads into tablets can be frequently problematic due to core fracture and cracking of the coat, which can result in the premature release of the active material from the dosage forms.

US5780055 discloses a tablet incorporating biologically active ingredient - loaded beads and cushioning beads comprising microcrystalline cellulose, wherein the cushioning bead is prepared by extrusion-spheronisation, followed by freeze-drying, and the cushioning bead has a diameter of about 0.2 to 2.0mm. These cushioning beads exhibit both brittle fracture and plastic deformation. Both brittle fracture and plastic deformation are desired because when the cushioning beads, mixed with biologically loaded beads are compacted, initial fragmentation into primary particles not only fills the voids between the biologically active ingredient - loaded beads, but also surrounds them. Plastic deformation would then enhance the particle-particle interactions, thereby producing stronger tablets.

EP 0824344 and EP 1075838 comprise a method of coating a pharmaceutical substrate, especially a tablet core, wherein a pharmaceutically acceptable powder coating material comprising active material is electrostatically applied to a surface of the substrate, wherein the coated substrate constitutes a dosage unit; and a powder coating material suitable for use in the electrostatic powder coating of a pharmaceutical substrate, in which the material is pharmaceutically acceptable, is treatable to form a film coating and includes composite particles, the composite particles comprising two or more components having different physical and/or chemical properties, the material comprising active material.

It is stated that when the powder material is first deposited on the tablet core it is in most cases only weakly adhered to the surface of the substrate and is easily dislodged. Treatment to form a film coating is especially advantageous when coating a pharmaceutical tablet core because the core itself is likely to be of low mechanical strength and the film coating can be used to impart strength and make the coated tablets more resistant to subsequent processing such as packaging and opening of packages.

It is further stated that in the coating process disclosed therein the tablet core is handled delicately throughout the coating process so that even a fragile tablet core is not damaged and the method may be employed to coat tablet cores that would be too fragile to withstand conventional tablet coating processes. Thus the method enables tablets of conventional shape but of a wider range of compositions to be produced; also, tablets of unconventional shapes, for example having opposite flat faces rather than conventional domed faces, may be produced by the invention. Such flat-faced tablets are generally too fragile to be coated using conventional methods.

The present invention provides an alternative method of making a pharmaceutical dosage form in which tablets having good structural integrity are obtained from a core which is formed under light compression. The structural integrity of the tablets can be measured by radial tensile strength. This is determined by diametrical compression measurement. The test can be carried out using a Schleuniger tester based on a counterweight principle. The tablet is placed between two anvils, a moving anvil driven by a speed-controlled electric motor presses the tablet against a stationary anvil. The maximal force which causes the tablet to fracture is then recorded and the radial tensile strength is calculated as follows:-

$$\sigma = 2F/D\,t\,\pi$$

where:

σ is tensile strength
F is maximal force to cause fracture during diametrical compression
D diameter
t thickness of tablet

According to the present invention there is provided a method of making a solid pharmaceutical dosage form comprising the steps of:

(i) forming a tablet core comprising a pharmaceutically active ingredient and one or more pharmaceutically acceptable adjuvants, the tablet core having a tensile strength of less than 38 N/cm$^2$.
(ii) electrostatically depositing a powder comprising fusible particles over at least 25% of the surface area of the tablet core and
(iii) heating the deposited powder to fuse the particles to form a coating film, thereby increasing the tensile strength of the dosage form, wherein the tablet core comprises two major opposing surfaces separated by a sidewall(s) and the powder is deposited over at least the major surfaces and at least a portion of the sidewalls(s) is not covered by the deposited powder.

The invention provides a means for obtaining tablets having good structural integrity which are formed from cores having a tensile strength of less than 38 N/cm$^2$ (2.5kP) i.e. cores that are so weak that previously they would have been regarded as too weak for practical use. The cores may have a tensile strength less than 30 N/cm$^2$ (2.0kP), preferably less than 22 N/cm$^2$ (1.5kP). The cores may be formed by light compression and enable coated components and fragile components, such as capsules, to be used within the compression blend with little or no damage.,

While EP 0824344 and EP 1075838 disclose the robustness of tablets may be improved by electrostatic coating of powder and fusing the references do not suggest that such weak tablet cores used in the present invention may be used to form viable pharmaceutical dosage forms.

The invention provides a simple effective means of improving the structural integrity of tablet cores by partially or fully coating the tablet core with a fusible powder and fusing the powder to form a film. In addition to improving the hardness the friability weight loss is significantly improved. The coating material may be selected to be readily soluble, gradually soluble or substantially insoluble in body fluids e.g. gastric juices, saliva etc. and thus the dosage form may be constructed to provide a rapidly disintegrating product or a sustained release product by suitable selection of coating materials.

The coating extending over the tablet core results from the deposition of a powder comprising fusible particles. This technique allows the formation of a thin, continuous film over surface areas of the tablet core. In general, the film will cover from 25 to 100% preferably 50 to 100% of the surface area of the tablet core. The resulting tablet preferably has a tensile strength of at least 50 N/cm$^2$, 60 N/cm$^2$ and most preferably at least 70 N/cm$^2$.

The shape of the tablet core is not critical since the deposition of powder can readily be achieved over a variety of shaped bodies. The tablet core may be formed by tableting techniques e.g. compression of powder and/or granules under light compression although other techniques such as moulding may be employed. A convenient tablet core has a circular cross-section and two major opposing surfaces which may be planar, for example planar with bevelled edge, concave, convex etc. The coating may conveniently extend over the major surfaces leaving the sidewall(s) exposed. Optionally the sidewall may be partially coated with the coating.

The tablet core comprises an adjuvant and a pharmaceutically active ingredient. The tablet core has a tensile strength of less than 38 N/cm$^2$, preferably less than 30 N/cm$^2$, more preferably less than 22 N/cm$^2$.

Generally the adjuvant will comprise a binder. Suitable binders are well known and include acacia, alginic acid, carboxymethylcellulose, hydroxyethylcellulose, hydroxypropylcellulose, dextrin, ethylcellulose, gelatin, glucose, guar gum, hydroxypropylmethylcellulose, magnesium aluminium silicate, kaltodectrin, methylcellulose, polyethylene oxide, povidone, sodium alginate and hydrogenated vegetable oils.

The tablet core may comprise a release rate controlling additive. For example, the drug may be held within a hydrophobic polymer matrix so that it is gradually leached out of the matrix upon contact with body fluids. Alternatively, the drug may be held within a hydrophilic matrix which gradually or rapidly dissolves in the presence of body fluid. The tablet core may comprise two or more layers having different release properties. The layers may be hydrophilic, hydrophobic or a mixture of hydrophilic and hydrophobic layers. Adjacent layers in a multilayer tablet core may be separated by an insoluble barrier layer or hydrophilic separation layer. An insoluble barrier layer may be formed of materials used to form the insoluble casing. A hydrophilic separation layer may be formed from a material more soluble than the other layers of the tablet core so that as the separation layer dissolves the release layers of the tablet core are exposed.

Suitable release rate controlling polymers include polymethacrylates, ethylcellulose, hydroxypropylmethylcellulose, methylcellulose, hydroxyethylcellulose, hydroxypropylcellulose, sodium carboxymethylcellulose, calcium carboxymethylcellulose, acrylic acid polymer, polyethylene glycol, polyethylene oxide, carrageenan, cellulose acetate, zein etc.

Suitable materials which swell on contact with aqueous liquids include polymeric materials include from cross-linked sodium carboxymethylcellulose, cross-linked hydroxypropylcellulose, high molecular weight hydroxypropylcellulose, carboxymethylamide, potassium methacrylatedivinylbenzene copolymer, polymethylmethacrylate, cross-linked polyvinylpyrrolidone and high molecular weight polyvinylalcohols.

The tablet core may comprise other conventional tableting ingredients, including diluents, disintegrants, lubricants, wetting agents, glidants, surfactants, release aids, colourants, gas producers, etc.

Suitable diluents include lactose, cellulose, dicalcium phosphate, sucrose, dextrose, fructose, xylitol, mannitol, sorbitol, calcium sulphate, starches, calcium carbonate, sodium carbonate, cellulose acetate, dextrates, dextrin, kaolin, lactitol, magnesium carbonate, magnesium oxide, maltitol, maltodextrin and maltose.

Suitable lubricants include magnesium stearate and sodium stearyl fumarate.

Suitable glidants include colloidal silica and talc.

Suitable wetting agents include sodium lauryl sulphate and docusate sodium.

A suitable gas producer is a mixture of sodium bicarbonate and citric acid.

The pharmaceutically active ingredient may be selected from a wide range of substances which may be administered orally. Suitable ingredients include acid-peptic and motility influencing agents, laxatives antidiarrhoeials, colorectal agents, pancreatic enzymes and bile acids, antiarrhythmics, antianginals, diuretics, anti-hypertensives, anti-coagulants, anti-thrombotics, fibrinolytics, haemostatics, hypolipidaemic agents, anti-anaemia and neurotropenia agents, hypnotics, anxiolytics, anti-psychotics, anti-depressants, anti-emetics, anti-convulsants, CNS stimulants, analgesics, anti-pyretics, anti-migraine agents, non-steroidal anti-inflammatory agents, anti-gout agents, muscle relaxants, neuro-muscular agents, steroids, hypoglycaemic agents, hyperglycaemic agents, diagnostic agents, antibiotics, anti-fungals, anti-malarials, anti-virals, immunosuppressants, nutritional agents, vitamins, electrolytes, anorectic agents, appetite suppressants, bronchodilators, expectorants, anti-tussives, mucolytic, decongestants, anti-glaucoma agents, oral contraceptive agents, diagnostic and neoplastic agents.

The pharmaceutical active ingredient may be present in beads, membrane coated beads or microcapsules. The membrane can provide a delayed release function when in contact with physiological fluid, which enables the masking of undesirable taste; a sustained or slow release of active; protection from gastric fluid; targeted release of the actives along the gastro-intestinal tract such as stomach, jejenum, duodenum and the colon. The membrane may comprise of any pharmaceutically acceptable materials. Suitable membrane forming ingredients may include acacia, albumin, modified cellulose native and modified starches, sugars, wax, acrylic and methacrylic polymers.

The powder forming the coating may be applied by any suitable technique e.g. spraying, fluidised bed, falling curtain and electrostatic spraying. Electrostatic application is preferred.

The electrostatic application of powder material to a substrate is known. Methods have already been developed in the fields of electrophotography and electrography and examples of suitable methods are described, for example, in Electrophotography and Development Physics, Revised Second Edition, by L.B. Schein, published by Laplacian Press, Morgan Hill California. The electrostatic application of powder material to a solid dosage form is known and techniques are disclosed, for example, in GB9929946.3, WO92/14451, WO96/35413, WO96/35516 and PCT/GB01/00425, and British Patent Application No. 9929946.3.

For example, WO92/14451 describes a process in which the cores of pharmaceutical tablets are conveyed on an earthed conveyor belt and electrostatically charged powder is deposited on the cores to form a powder coating on the surface of the cores.

A powder material for electrostatic application to a substrate should have certain properties. For example, the electrical properties of the powder material should be such as to make the powder material suitable for electrostatic application, and other properties of the powder material should be such that the material can be secured to the substrate once electrostatic application has taken place.

WO96/35413 describes a powder material which is especially suitable for electrostatic application to a poorly-conducting (non-metal) substrate such as a pharmaceutical tablet. Because it may be difficult to find a single component capable of providing the material with all the desired properties, the powder material comprises a number of different components which together are capable of providing the material with all or at least as many as possible of the desired properties, the components being co-processed to form "composite particles". For example, the powder material may comprise composite particles including one component which is fusible to form a continuous film on the surface of the substrate, and another component which has desirable electrical properties.

A potential disadvantage of the above mentioned powder materials, however, is that they are not readily adaptable to changes in formulation. The formulation of a powder material may be changed for a number of different reasons. For example, if the material is a coloured material, there may be a change in the colourant, or if the material is an active material, for example a physiologically active material there may be a change in the type of active material, or in the concentration of that active material. Because all the components of the powder material are intimately mixed, any change in the components will alter the material's electrical properties and hence its performance in electrostatic application. Whenever there is a change in formulation, it may therefore be necessary, for optimum performance, to adjust the content of the component(s) that make the material suitable for electrostatic application, or perhaps even to use a different component.

PCT/GB01/00425 discloses a method of electrostatically applying a powder material to a substrate, wherein at least some of the particles of the material comprise a core and a shell surrounding the core, the core and the shell having different physical and/or chemical properties.

Where the particles of the powder material comprise a core and a shell surrounding the core, it is possible to place those components which are likely to be altered, for example colourant in the core, and to provide a more universal shell composition which is suitable for use with various core compositions, so that alterations may be made to the components that are in the core without substantially affecting the overall suitability of the powder material; thus, the shell ensures that the change in composition of the core does not affect the performance of the material in electrostatic application. Accordingly, alterations to one component of the powder material may be made with minimum alteration in the amounts of other components.

Generally, the powder material includes a component which is fusible, and that component may be present in the shell or in the core or in both the shell and the core. Advantageously, the fusible component is treatable to form a continuous film coating. Examples of suitable components are as follows: polyacrylates, for example polymethacrylates; polyesters; polyurethanes; polyamides, for example nylons; polyureas; polysulphones; polyethers; polystyrene; polyvinylpyrrolidone; biodegradable polymers, for example polycaprolactones, polyanhydrides, polylactides, polyglycolides, polyhydroxybutyrates and polyhydroxyvalerates; polyols, for example lactitol, sorbitol xylitol, galactitol and maltitol; sugars, for example sucrose, dextrose, fructose, xylose and galactose; hydrophobic waxes and oils, for example vegetable oils and hydrogenated vegetable oils (saturated and unsaturated fatty acids) e.g. hydrogenated castor oil, carnauba wax, and beeswax; hydrophilic waxes; polyalkenes and polyalkene oxides; polyethylene glycol. Clearly there may be other suitable materials, and the above are given merely as examples. One or more fusible materials may be present. Preferred fusible materials generally function as a binder for other components in the powder.

In general the powder material should contain at least 30%, usually at least 35%, advantageously at least 80%, by weight of material that is fusible, and, for example, fusible material may constitute up to 95%, e.g. up to 85%, by weight of the powder. Wax, if present, is usually present in an amount of no more than 6%, especially no more than 3% by weight, and especially in an amount of at least 1% by weight, for example 1 to 6%, especially to 1 to 3%, by weight of the powder material.

Of the materials mentioned above, polymer binders (also referred to as resins) should especially be mentioned. Examples include polyvinylpyrrolidone, hydroxypropyl cellulose, hydroxypropyl methylcellulose phthalate, hydroxypropyl methylcellulose acetate succinate and methacrylate polymers, for example an ammonio-methacrylate copolymer, for example those sold under the name Eudragit.

Often resin will be present with a wax as an optional further fusible component in the core; the presence of a wax may, for example, be useful where fusing is to take place by a contact system for example using a heated roller, or where it is desired to provide a glossy appearance in the fused film. The fusible component may comprise a polymer which is cured during the treatment, for example by irradiation with energy in the gamma, ultra violet or radio frequency bands. For example, the core may comprise thermosetting material which is liquid at room temperature and which is hardened after application to the substrate.

Preferably, the powder material includes a material having a charge-control function. That functionality may be incorporated into a polymer structure, as in the case of Eudragit resin mentioned above, and/or, for a faster rate of charging, may be provided by a separate charge-control additive. Material having a charge-control function may be present in the shell or in the core or in both shell and core. Examples of suitable charge-control agents are as follows: metal salicylates, for example zinc salicylate, magnesium salicylate and calcium salicylate; quaternary ammonium salts; benzalkonium chloride; benzethonium chloride; trimethyl tetradecyl ammonium bromide (cetrimide); and cyclodextrins and their adducts. One or more charge-control agents may be used. Charge-control agent may be present, for example, in an amount of up to 10% by weight, especially at least 1 % by weight, for example from 1 to 2% by weight, based on the total weight of the powder material.

The powder material may also include a flow aid. The flow aid reduces the cohesive and/or other forces between the particles of the material to improve the flowability of the powder. Suitable flow aids (which are also known as "surface additives") are, for example, as follows: colloidal silica; metal oxides, e.g. fumed titanium dioxide, zinc oxide or alumina; metal stearates, e.g. zinc, magnesium or calcium stearate; talc; functional and non-functional waxes, and polymer beads, e.g. poly-methyl methacrylate beads, fluoropolymer beads and the like. Such materials may also enhance tribocharging. A mixture of flow aids, for example silica and titanium dioxide, should especially be mentioned. The powder material may contain, for example, 0 to 3% by weight, advantageously at least 0.1 %, e.g. 0.2 to 2.5%, of surface additive flow aid.

Often the powder material includes a colourant and/or an opacifier. When the powder comprises a core and shell such components are preferably present in the core. Examples of suitable colourants and opacifiers are as follows: metal oxides, e.g. titanium dioxide, iron oxides; aluminium lakes, for example, indigo carmine, sunset yellow and tartrazine; approved food dyes; natural pigments. A mixture of such materials may be used if desired. Opacifier preferably constitutes no more than 50%, especially no more than 40%, more especially no more than 30%, for example no more than 10% by weight of the powder material, and may be used, for example, in an amount of at least 5% by weight of the powder. Titanium dioxide is an especially useful opacifier, providing white colour and having good hiding power and tinctorial strength.

Colourant present with opacifier may, for example, constitute no more than 10%, preferably from 1 to 5%, by weight of the powder. If there is no opacifier, the colourant may be, for example, 1 to 15%, e.g. 2 to 15%, especially 2 to 10%, by weight of the powder. To achieve optimum colour, amounts of up to 40% by weight of colourant may be needed in some cases, for example if inorganic pigments, e.g. iron oxides, are used. However, the powder material usually contains, for example, from 0 to 25% by weight in total of colourant and/or opacifier.

The powder material may also include a dispersing agent, for example a lecithin. The dispersing agent is preferably present with the colourant/opacifier (that is, preferably in the core), serving to improve the dispersion of the colourant and opacifier, more especially when titanium dioxide is used. The dispersing component is preferably a surfactant which may be anionic, cationic or non-ionic, but may be another compound which would not usually be referred to as a "surfactant" but has a similar effect. The dispersing component may be a co-solvent. The dispersing component may be one or more of, for example, sodium lauryl sulphate, docusate sodium, Tweens (sorbitan fatty acid esters), polyoxamers and cetostearyl alcohol. Preferably, the powder material includes at least 0.5%, e.g. at least 1%, for example from 2% to 5%, by weight of dispersing component, based on the weight of the powder material. Most often it is about 10% by weight of the colourant and opacifier content.

The powder material may also include a plasticiser, if necessary, to provide appropriate rheological properties. A plasticiser may be present in the core and/or the shell, but usually, if present, a plasticiser is included with resin used for the core to provide appropriate rheological properties, for example for preparation of the core by extrusion in a melt extruder. Examples of suitable plasticisers include polyethylene glycols, triethyl citrate, acetyltributyl citrate, acetyltriethyl citrate, tributyl citrate, diethyl phthalate, dibutyl phthalate, dimethyl phthalate, dibutyl sebacate and glyceryl monostearate.

A plasticiser may be used with a resin in an amount, for example, of up to 50% by weight of the total of that resin and plasticiser, the amount depending inter alia on the particular plasticisers used. The powder may contain an amount of up to 50% by weight of plasticiser.

The powder coating material may further include one or more taste modifiers, for example aspartame, acesulfame K, cyclamates, saccharin, sugars and sugar alcohols or flavourings. Preferably there is no more than 5%, more preferably no more than 1%, of flavouring based on the weight of the powder material, but larger or smaller amounts may be appropriate, depending on the particular taste modifier used.

If desired the powder material may further include a filler or diluent. Suitable fillers and diluents are essentially inert and low cost materials with generally little effect on the colour or other properties of the powder. Examples are as follows: alginic acid; bentonite; calcium carbonate; kaolin; talc; magnesium aluminium silicate; and magnesium carbonate.

The particle size of the powder material has an important effect on the behaviour of the material in electrostatic application. Although materials having a small particle size are recognised as having disadvantages such as being more difficult to produce and to handle by virtue of the material's cohesiveness, such material has special benefits for electrostatic application and the benefits may more than counter the disadvantages. For example, the high surface to mass ratio provided by a small particle increase the electrostatic forces on the particle in comparison to the inertial forces. Increasing the force on a particle has the benefit of increasing the force that causes it to move into contact with the substrate, whilst a reduction in the inertia reduces the force needed to accelerate a particle and reduces the likelihood of a particle arriving at the substrate bouncing back off the substrate. However, very small particle sizes may not be achievable where the coating material comprises a high proportion of a particular ingredient, for example a high proportion of active material.

Preferably, at least 50% by volume of the particles of the material have a particle size no more than $100\mu$m. Advantageously, at least 50% by volume of the particles of the material have a particle size in the range of $5\mu$m to $40\mu$m. More advantageously, at least 50% by volume of the particles of the material have a particle size in the range of 10 to $25\mu$m.

Powder having a narrow range of particle size should especially be mentioned. Particle size distribution may be quoted, for example, in terms of the Geometric Standard Deviation ("GSD") ratios $d_{90}/d_{50}$ or $d_{50}/d_{10}$ where $d_{90}$ denotes the particle size at which 90% by volume of the particles are below this figure (and 10% are above), $d_{10}$ represents the particle size at which 10% by volume of the particles are below this figure (and 90% are above), and $d_{50}$ represents the mean particle size. Advantageously, the mean ($d_{50}$) is in the range of from 5 to $40\mu$m, for example, from 10 to $25\mu$m. Preferably, $d_{90}/d_{50}$ is no more than 1.5, especially no more than 1.35, more especially no more than 1.32, for example in the range of from 1.2 to 1.5, especially 1.25 to 1.35, more especially 1.27 to 1.32, the particle sizes being measured, for example, by Coulter Counter or a laser particle size analyser. Thus, for example, the powder may have $d_{50} = 10\mu$m, $d_{90} = 13\mu$m, $d_{10} = 7\mu$m, so that $d_{90}/d_{50} = 1.3$ and $d_{50}/d_{10} = 1.4$.

The powder material is fusible so that it is treatable to form a continuous film coating.

It is important that the powder can be fused or treated without degradation of any active material in the powder and without degradation of the tablet core. For some materials it may be possible for the treatment step to involve temperatures up to and above 250°C. Preferably, however, the powder material is fusible at a pressure of less than 100lb/sq. inch, preferably at atmospheric pressure, at a temperature of less than 200°C, and most commonly below 150°C, and often at least 80°C, for example in the range of from 100 to 140°C

Fusing of the powder material may be carried out by any of a number of different fusing methods. The powder material is preferably fused by changing the temperature of the powder, for example by radiant fusing using electromagnetic radiation, for example infra red radiation or ultra-violet radiation, or conduction or induction, or by flash fusing. The amount of heat required may be reduced by applying pressure to the powder material, for example by cold pressure fusing or hot roll fusing.

Preferably, the powder material has a glass transition temperature (Tg) in the range of 40°C to 120°C. Advantageously, the material has a Tg in the range of 50°C to 100°C. A preferred minimum Tg is 55°C, and a preferred maximum Tg is 70°C. Accordingly, more advantageously, the material has a Tg in the range of 55°C to 70°C. Generally, the powder material should be heated to a temperature above its softening point, and then allowed to cool to a temperature below its Tg.

If the dosage form is a rapid disintegrating tablet, the film formed by the powder material must be readily soluble in water.

The invention will now be described with reference to the accompanying drawing in which:

Figures 2 and 3 represent cross section through tablets made in accordance with the invention,

Figure 4 represents a plot of weight loss against revolution for tablet cores and coated tablets tested in a friability tester and

Figure 5 represents a plot of weight loss against time for tablet cores and coated tablets tested on a shaker.

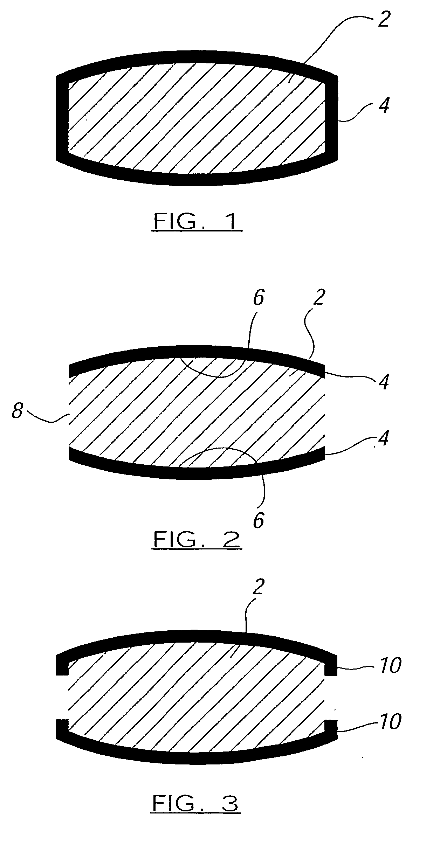

Figure 1 shows a tablet core 2 in the form of a circular biconvex tablet. The core is completely coated with a fused film 4 [Reference example].

Figure 2 illustrates a tablet core 2 of the same configuration as that in Figure 1. Coating 4 is applied to the two major surfaces 6 leaving the sidewall 8 uncovered. The coating provides complete protection to the major surfaces and limited protection to the edges of the tablet core.

Figure 3 shows a similar arrangement to Figure 2 with the coating 4 extending slightly along the sidewall 8 that regions 10 to provide additional protection to the edges of the tablet.

In the embodiments of Figures 2 and 3 different coatings may be applied to the major surfaces of the table core.

The invention will now be described with reference to the following Examples.

Example 1

A tablet core was formed by distribution of 20g a 5% w/w aqueous citric acid solution over a mixture of 360g of mannitol (Perlitol™) and 20g of sodium starch glycollate (Explotab™) using a planetary mixer (Kenwood Magimix 4100) and drying the resulting damp powder a forced air oven. The dried powder was blended with 4% cross-linked PVP (polyvinylpyrrolidine), Plolyplasdone XL-10, 1 % of magnesium stearate, 0.5% aspartame and 0.1% lemon flavour and lightly compressed using 10mm diameter punches to give biconvex tablets of approximately 230mg by a Manesty F3 press.

A coat formulation was prepared by blending 68.6% PVP-VA copolymer, 10% methacrylic acid copolymer 4.4% PEG3000, 4.5% xylitol, 10% titanium dioxide and 2.5% ponceau 4R lake, melt extrusion of the mix using a EuroLab Extruder and micronisation of the extrudate.

The coat was applied by electrostatic deposition to each face of the table as in Figure 2 and the applied powder was fused using hot air at 160°C for 90 seconds. The fused coat was approximately 50μm thick.

The structural integrity of the tablets can be measured by radial tensile strength. This is determined by diametrical compression measurement. The test was carried out using a Schleuniger tester based on a counterweight principle. The tablet is placed between two anvils, a moving anvil driven by a speed-controlled electric motor presses the tablet against a stationary anvil. The maximal force which causes the tablet to fracture is then recorded and the radial tensile strength is calculated as described previously.

Tablet friability was determined according to standard US Pharmacopoeia method using a Copley friability tester. The tablets were weighed (6.5g) placed in a drum with an internal diameter between 283 and 291 mm and a depth between 36 and 40mm. One side of the drum is removable. The tablets were tumbled at each turn of the drum by a curved projection with an inside radius between 75.5 and 85.5 that extends from the middle of the drum to the outer wall. The drum is attached to the horizontal axis of a device that rotates at $25 \pm 1$rpm. At each turn, the tablets roll or slide and fall onto the drum wall or onto each other. After 100 revolutions i.e. 4 minutes, the intact tablets were collected, weighed and percentage weight loss (friability) was then calculated.

The properties of the tablet cores and coated tablets were as follows:

|  | Cores | Coated Tablets |
| --- | --- | --- |
| tensile strength | 18 N/cm$^2$ | 40 N/cm$^2$ |
| friability weight loss | 0.6% | 0.8% |
| oral disintegration | 16 seconds | 22 seconds |

The Example demonstrates a significant increase in tensile strength after the application of fusible coating.

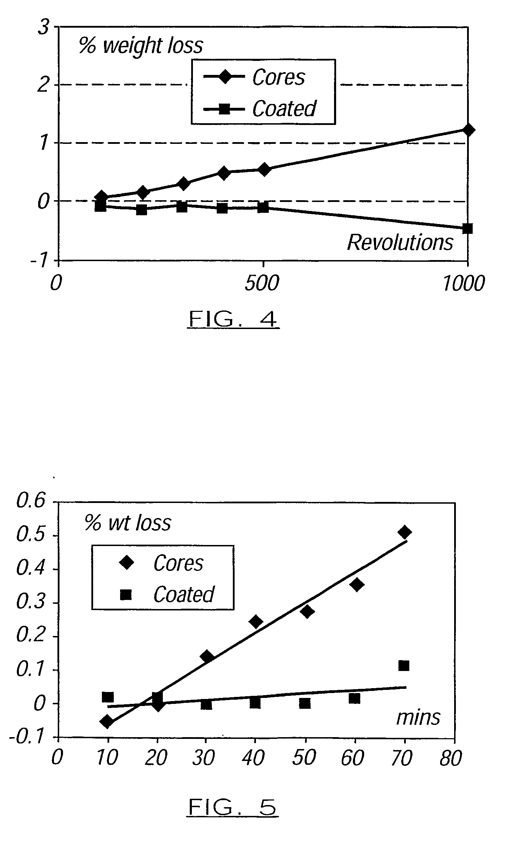

During tablet manufacture and packaging, the surface of tablets may become eroded as they slide along the production line. This problem can become acute if the tablets are fragile and soft. A modified friability test was carried out by inclining the friability tester at 30° to the horizontal to give an indication of tablet erosion. Weight loss after up to 1000 revolutions was determined on both coated and uncoated tablets and the results are shown in Figure 4.

It is evident from Figure 4 that the uncoated tablets (tablet cores) erode as the test proceeds whereas the coated tablets slightly increase in weight as they pick up atmospheric moisture.

In another test for robustness, tablets were placed in a polypropylene container and shaken on the base of a Fritsch sieve shaker. This test is intended to simulate the shaking that tablets might experience when stored in a bottle or similar container. The weight loss of the tablets was measured at 10 minutes intervals and results are shown in Figure 5. The results again demonstrate the improved robustness of the tablets of this invention.

Example 2 [Reference example]

Tablet cores were prepared by wet granulation of mannitol Perlitol™ (612g), microcrystalline cellulose Vivapur™ (200g), maize starch Aci-di-sol™ (50g), croscarmellose sodium Explotab™ (50g), sodium starch glycollate (50g), sodium lauryl sulphate (2g) with an aqueous solution of citric acid (30g), and the resulting wet mass was dried and passed through a 1 mm screen. Magnesium stearate (5g) and colloidal silicon dioxide (1g) were blended into the screened granules, and then this blend (194g) was further blended with talc (6g). The resulting blend was lightly compressed on a Manesty F3 machine fitted with 10mm concave tooling.

Two coat formulations were prepared as follows.

Coat formulation A was prepared by blending 64.5% PVP-VA copolymer, 20% methacrylic acid copolymer 3% PEG3000, 10% titanium dioxide and 2.5% ponceau 4R lake, melt extrusion of the mix and micronisation of the extrudate.

Coat formulation B was prepared by blending 58.5% PVP-VA copolymer, 20% methacrylic acid copolymer, 9% PEG3000, 10% titanium dioxide and 2.5% ponceau 4R lake, melt extrusion of the mix and micronisation of the extrudate.

The tablets were coated as in Figure 1 by electrostatic deposition of coat A on one side of the tablet and fusing with air at 175°C for 180 seconds followed by electrostatic deposition of coat B on the second side of the tablet and fusing with air at 135°C for 270 seconds. The coats were approximately 50 microns thick.

The properties of the tablet cores and coated tablets were as follows:

|  | Cores | Coated Tablets |
| --- | --- | --- |
| tensile strength | 17 Ncm$^2$ | 70 N/cm$^2$ |
| friability weight loss | 1.5% | 0.8% |

Example 3

A soft tablet was prepared by combining two pre-prepared granules with other ancillary ingredients in a Y-cone blender, then compressed on a Manesty F3 machine with 10mm round concaved tooling. The tablet formulation is as follows:

| | |
| --- | --- |
| Granule A: | 1114.5g |
| Granule B: | 360.0g |
| Aspartame: | 7.5g |
| Lemon flavour: | 3.0g |
| Magnesium stearate: | 15.0g |
| The formation for granule A is: | |
| Mannitol (Perlitol™) | 2730g |
| Sodium starch glycollate (Explotab™) | 120.0g |
| Citric acid | 75.0g |
| Lactitol | 75.0g |

Citric acid and lactitol were dissolved in demineralised water to make a granulation solution , which was then used to granulate mannitol and sodium starch glycollate in a Diosna mixer. The wet mass was then dried in a Niro fluid bed drier at 60°C.

The formulation for granule B is:

| | |
| --- | --- |
| Powdered mannitol | 600.0g |
| Citric acid | 5.0g |
| Lactitol | 75.0g |
| Crospovidone (Polyplasdone™) | 250.0g |

Citric acid and lactitol were dissolved in demineralised water to make a granulation solution. Powdered mannitol and crospovidone were granulated in a planetary mixer and dried in a forced air oven at 60°C.

A coating formulation was prepared by blending 68.6% PVP-VA, 10% Eudragit™ (methacrylic acid copolymer), 4.4% PEG, 10% titanium dioxide, 4.5% xylitol and 2.5% ponceau 4R lake, melt extrusion of the mix and micronisation of the extrudate.

The coat was applied to the core by electrostatic deposition of the coating to the top and bottom of the tablet as shown in Figure 2. Variable weight of coating was applied to the core.

The properties of the tablets were as follows:

| | | | |
| --- | --- | --- | --- |
| Coating thickness | 0 microns (no coating) | 28 microns | 69 microns |
| Tensile strength | 29 N/cm$^2$ | 58 N/cm$^2$ | 62 N/cm$^2$ |
| Friability (USP) | 0.7% | 0.4% | 0.5% |

Example 4

The same core and coat formulations as in Example 3 were used to prepare coated tablets except that the tablet cores were prepared at lower hardness using the Manesty F3 tablet press. The coating was applied to the top and bottom of the tablet core by electrostatic coating and followed by fusion using hot air at 150°C for 90 seconds. The coating thickness was approximately 50 microns. The improvement in the integrity of the tablets of this invention is demonstrated by the increases in tensile strength as shown below:

|  | Uncoated tablet core | Coated and fused tablet |
|---|---|---|
| Tablet one | 14 N/cm$^2$ | 51 N/cm$^2$ |
| Tablet two | 21 N/cm$^2$ | 63 N/cm$^2$ |

Example 5

The same core formulations as in Examples 3 and 4 were used except that coatings of xylitol (Xylitab™) were applied onto the top and bottom of tablet core by compression coating using a Manesty F3 tablet press. The applied coatings were fused using hot air at 120°C for 90 seconds on each side. The improvement in the integrity of the tablets of this invention is demonstrated by the significant increase in tensile strength as shown below:

|  | Unfused | Fused |
|---|---|---|
| Tensile strength | 20 N/cm$^2$ | 58 N/cm$^2$ |

**Claims**

1. A method of making a solid pharmaceutical dosage form comprising the steps of:

> (i) forming a tablet core comprising a pharmaceutically active ingredient and one or more pharmaceutically acceptable adjuvants, the tablet core having a tensile strength of less than 38 N/cm$^2$,
> (ii) electrostatically depositing a powder comprising fusible particles over at least 25% of the surface area of the tablet core and
> (iii) heating the deposited powder to fuse the particles to form a coating film, thereby increasing the tensile strength of the dosage form, wherein the tablet core comprises two major opposing surfaces separated by a sidewall(s) and the powder is deposited over at least the major surfaces and at least a portion of the sidewalls (s) is not covered by the deposited powder.

2. A method as claimed in Claim 1 in which the tablet core is formed by compression of powder ingredients.

3. A method as claimed in any preceding Claim in which the tensile strength of the tablet core before depositing and heating the powder is less than 22 N/cm$^2$.

4. A method as claimed in any preceding claim in which the pharmaceutical dosage form has a tensile strength of at least 60 N/cm$^2$.

5. A method as claimed in any preceding claim in which the tablet core has a circular cross-section and the two major opposing surfaces are convex

6. A method as claimed in any preceding claim in which the tablet core comprises a binder selected from acacia, alginic acid, carboxymethylcellulose, hydroxyethylcellulose, hydroxypropylcellulose, dextrin, ethylcellulose, gelatin, glucose, guar gum, hydroxypropylmethylcellulose, magnesium aluminium silicate, Maltodextrin, methylcellulose, polyethylene oxide, povidone, sodium alginate and hydrogenated vegetable oils.

7. A method as claimed in any preceding Claim in which the tablet core additionally comprises a release rate controlling polymer selected from polymethacrylates, ethylcellulose, hydroxypropylmethylcellulose, methylcellulose, hydroxyethylcellulose, hydroxypropylcellulose, sodium carboxymethylcellulose, calcium carboxymethylcellulose, acrylic acid polymer, polyethylene glycol, polyethylene oxide, carrageenan, cellulose acetate, glyceryl monostearate and zein.

8. A method as claimed in any preceding Claim in which the tablet core comprises a hydrophobic matrix containing an active ingredient or a hydrophilic matrix containing an active ingredient.

9.  A method as claimed in any preceding claim in which the tablet core is rapidly soluble or rapidly disintegratable.

10. A method as claimed in any preceding claim in which the active ingredient is selected from acid-peptic and motility influencing agents, laxatives, antidiarrhoeials, colorectal agents, pancreatic enzymes and bile acids, antiarrhythmics, antianginals, diuretics, anti-hypertensives, anti-coagulants, anti-thrombotics, fibrinolytics, haemostatics, hypolipidaemic agents, anti-anaemia and neurotropenia agents, hypnotics, anxiolytics, anti-psychotics, anti-depressants, anti-emetics, anti-convulsants, CNS stimulants, analgesics, anti-pyretics, anti-migraine agents, non-steroidal anti-inflammatory agents, anti-gout agents, muscle relaxants, neuro-muscular agents, steroids, hypoglycaemic agents, hyperglycaemic agents, diagnostic agents, antibiotics, anti-fungals, anti-malarials, anti-virals, immunosuppressants, nutritional agents, vitamins, electrolytes, anorectic agents, appetite suppressants, bronchodilators, expectorants, anti-tussives, mucolytes, decongestants, anti-glaucoma agents, oral contraceptive agents, diagnostic and neoplastic agents.

11. A method as claimed in Claim 8 in which the beads, membrane coated beads or microcapsules have a particle size in the range 50 to 1500 $\mu$m.

12. A method as claimed in Claim 9 in which the membrane has a function selected from taste masking function, an enteric protection function, a sustained release function to allow the release of an active from the dosage form over a sustained period of time and a controlled release function to allow the release of an active at targeted site along the gastrointestinal tract.

13. A method as claimed in any preceding Claim in which the tablet core comprises a polymeric material which swells on contact with aqueous liquid, selected from cross-linked sodium carboxymethylcellulose, cross-linked hydroxypropylcellulose, high molecular weight hydroxypropylcellulose, carboxymethylamide, potassium methacrylatedivinylbenzene copolymer, polymethylmethacrylate, cross-linked polyvinylpyrrolidone and high molecular weight polyvinylalcohols.

14. A method as claimed in any preceding Claim in which the coating is rapidly soluble in water.

15. A method as claimed in any preceding Claim in which the coating comprises a polymer resin selected from polymethacrylates, cellulose and its derivatives, cellulose ethers and esters and cellulose acetate phthalate.

**Patentansprüche**

1.  Verfahren zum Herstellen einer festen pharmazeutischen Darreichungsform, umfassend die Schritte des:

    (i) Erzeugens eines Tablettenkerns, umfassend einen pharmazeutischen Wirkstoff und einen oder mehrere pharmazeutisch verträgliche Hilfsstoffe, wobei der Tablettenkern eine Bruchfestigkeit von weniger als 38 N/cm$^2$ aufweist,
    (ii) elektrostatischen Aufbringens eines Pulvers, umfassend schmelzbare Teilchen, auf mindestens 25% der Oberfläche des Tablettenkerns und
    (iii) Erwärmens des aufgebrachten Pulvers, um die Teilchen zu schmelzen, um einen Beschichtungsfilm zu erzeugen, wodurch die Bruchfestigkeit der Darreichungsform erhöht wird, wobei der Tablettenkem zwei entgegengesetzte Hauptoberflächen umfasst, die durch eine Seitenwand/Seitenwände getrennt sind, und das Pulver auf mindestens den Hauptoberflächen aufgebracht ist und mindestens ein Teil der Seitenwand/Seitenwände nicht mit dem aufgebrachten Pulver bedeckt ist.

2.  Verfahren nach Anspruch 1, wobei der Tablettenkem durch Zusammenpressen der Pulverbestandteile erzeugt wird.

3.  Verfahren nach einem der vorangegangenen Ansprüche, wobei die Bruchfestigkeit des Tablettenkerns vor dem Aufbringen und Erwärmen des Pulvers weniger als 22 N/cm$^2$ beträgt.

4.  Verfahren nach einem der vorangegangenen Ansprüche, wobei die pharmazeutische Darreichungsform eine Bruchfestigkeit von mindestens 60 N/cm$^2$ aufweist.

5.  Verfahren nach einem der vorangegangenen Ansprüche, wobei der Tablettenkern einen kreisförmigen Querschnitt aufweist und die beiden entgegengesetzten Hauptoberflächen konvex sind.

**6.** Verfahren nach einem der vorangegangenen Ansprüche, wobei der Tablettenkern ein Bindemittel umfasst, ausgewählt aus Akaziengummi, Alginsäure, Carboxymethylcellulose, Hydroxyethylcellulose, Hydroxypropylcellulose, Dextrin, Ethylcellulose, Gelatine, Glukose, Guarmehl, Hydroxypropylmethylcellulose, Magnesiumaluminiumsilikat, Maltodextrin, Methylcellulose, Polyethylenoxid, Povidon, Natriumalginat und hydrierten Pflanzenölen.

**7.** Verfahren nach einem der vorangegangenen Ansprüche, wobei der Tablettenkern zusätzlich ein die Freisetzungsrate regulierendes Polymer umfasst, ausgewählt aus Polymethacrylaten, Ethylcellulose, Hydroxypropylmethylcellulose, Methylcellulose, Hydroxyethylcellulose, Hydroxypropylcellulose, Natriumcarboxymethylcellulose, Calciumcarboxymethylcellulose, Acrylsäurepolymer, Polyethylenglykol, Polyethylenoxid, Carrageenan, Celluloseacetat, Glycerylmonostearat und Zein.

**8.** Verfahren nach einem der vorangegangenen Ansprüche, wobei der Tablettenkern eine hydrophobe Matrix, die einen Wirkstoff enthält, oder eine hydrophile Matrix, die einen Wirkstoff enthält, umfasst.

**9.** Verfahren nach einem der vorangegangenen Ansprüche, wobei der Tablettenkern schnell löslich oder schnell zerfallbar ist.

**10.** Verfahren nach einem der vorangegangenen Ansprüche, wobei der Wirkstoff ausgewählt ist aus säureverdauungsfördernden und motilitätbeeinflussenden Mitteln, Abführmitteln, Antidiarrhoika, Kolorektalmitteln, pankreatischen Enzymen und Gallensäuren, Antiarrhythmika, antiangiösen Mitteln, Diuretika, Antihypertensiva, Antikoagulanzien, Antithrombotika, Fibrinolytika, Hämatostatika, Hypolipidämiemitteln, Antianämiemitteln und Neurotropika, Hypnotika, Anxiolytika, Neuroleptika, Antidepressiva, Antiemetika, Antikonvulsiva, ZNS-Stimulanzien, Analgetika, Antipyretika, Antimigränemitteln, nichtsteroidalen Antiphlogistika, Antigichtmitteln, Muskelrelaxanzien, neuromuskulären Mitteln, Steroiden, hypoglykämischen Mitteln, hyperglykämischen Mitteln, diagnostischen Mitteln, Antibiotika, Fungiziden, Antimalariamitteln, Viruziden, Immunsuppressiva, Nahrungsmitteln, Vitaminen, Elektrolyten, Anorektika, Appetitzügler, Bronchospasmolytika, Expektoranzien, Antitussiva, Mukolytika, abschwellenden Mitteln, Antiglaukommitteln, oralen Kontrazeptiva, diagnostischen und neoplastischen Mitteln.

**11.** Verfahren nach Anspruch 8, wobei die Kügelchen, membranbeschichteten Kügelchen oder Mikrokapseln eine Teilchengröße im Bereich von 50 bis 1500 $\mu$m aufweisen.

**12.** Verfahren nach Anspruch 9, wobei die Membran eine Funktion aufweist, ausgewählt aus geschmackmaskierender Funktion, Darmschutzfunktion, Funktion verlängerter Freisetzung, um die Freisetzung eines Wirkstoffs aus der Darreichungsform über einen verlängerten Zeitraum zu erlauben, und Funktion regulierter Freisetzung, um die Freisetzung eines Wirkstoffs an der zielgerichteten Stelle entlang des Gastrointestinaltrakts zu erlauben.

**13.** Verfahren nach einem der vorangegangenen Ansprüche, wobei der Tablettenkem ein polymeres Material umfasst, welches bei Kontakt mit wässriger Flüssigkeit quellt, ausgewählt aus vernetzter Natriumcarboxymethylcellulose, vernetzter Hydroxypropylcellulose, Hydroxypropylcellulose hohen Molekulargewichts, Carboxymethylamid, methacryliertes Kaliumdivinylbenzolcopolymer, Polymethylmethacrylat, vernetztem Polyvinylpyrrolidon und Polyvinylalkohole hohen Molekulargewichts.

**14.** Verfahren nach einem der vorangegangenen Ansprüche, wobei die Beschichtung in Wasser schnell löslich ist.

**15.** Verfahren nach einem der vorangegangenen Ansprüche, wobei die Beschichtung ein Polymerharz umfasst, ausgewählt aus Polymethacrylaten, Cellulose und ihren Derivaten, Celluloseethern und -estern und Celluloseacetatphthalat.

**Revendications**

**1.** Procédé de fabrication d'une forme posologique pharmaceutique solide comprenant les étapes consistant à :

(i) former un noyau de comprimé comprenant un principe actif sur le plan pharmaceutique et un ou plusieurs adjuvants acceptables sur le plan pharmaceutique, le noyau de comprimé ayant une résistance à la traction inférieure à 38 N/cm$^2$,
(ii) déposer par effet électrostatique une poudre comprenant des particules fusibles sur au moins 25% de la surface du noyau de comprimé, et

(iii) chauffer la poudre déposée pour fondre les particules en vue de former une pellicule protectrice, augmentant ainsi la résistance à la traction de la forme posologique, où le noyau de comprimé comprend deux surfaces principales opposées séparées pour une ou des parois latérales et la poudre est déposée sur au moins la surface principale et au moins une partie de la ou des parois latérales n'est pas recouverte par la poudre déposée.

2. Procédé selon la revendication 1, dans lequel le noyau de comprimé est formé par compression d'ingrédients en poudre.

3. Procédé selon l'une quelconque des revendications précédentes, dans lequel la résistance à la traction du noyau de comprimé avant de déposer et chauffer la poudre, est inférieure à 22 N/cm$^2$.

4. Procédé selon l'une quelconque des revendications précédentes, dans lequel la forme posologique pharmaceutique présente une résistance à la traction d'au moins 60 N/cm$^2$.

5. Procédé selon l'une quelconque des revendications précédentes, dans lequel le noyau de comprimé comporte une coupe droite circulaire et les deux surfaces principales opposées sont convexes.

6. Procédé selon l'une quelconque des revendications précédentes, dans lequel le noyau de comprimé comprend un liant choisi parmi la gomme arabique, l'acide alginique, la carboxyméthylcellulose, l'hydroxyéthylcellulose, l'hydroxypropylcellulose, la dextrine, l'éthylcellulose, la gélatine, le glucose, la gomme de guar, l'hydroxypropyméthylcellulose, le silicate de magnésium et d'aluminium, la maltodextrine, la méthylcellulose, le poly(oxyde d'éthylène), la povidone, l'alginate de sodium et les huiles végétales hydrogénées.

7. Procédé selon l'une quelconque des revendications précédentes, dans lequel le noyau de comprimé comprend en outre un polymère régulant la vitesse de libération choisi parmi les polyméthacrylates, l'éthylcellulose, l'hydroxypropylméthylcellulose, la méthylcellulose, l'hydroxyéthylcellulose, l'hydroxypropylcellulose, la carboxyméthylcellulose sodique, la carboxyméthylcellulose calcique, un polymère d'acide acrylique, le polyéthylèneglycol, le poly(oxyde d'éthylène), la carraghénine, l'acétate de cellulose, le monostéarate de glycéryle et la zéine.

8. Procédé selon l'une quelconque des revendications précédentes, dans lequel le noyau de comprimé comprend une matrice hydrophobe contenant un principe actif ou une matrice hydrophile contenant un principe actif.

9. Procédé selon l'une quelconque des revendications précédentes, dans lequel le noyau de comprimé est rapidement soluble ou peut rapidement se désintégrer.

10. Procédé selon l'une quelconque des revendications précédentes, dans lequel le principe actif est choisi parmi l'acide peptique et les agents influençant la motilité, les laxatifs, les antidiarrhéiques, les agents recto-coliques, les enzymes pancréatiques et les acides biliaires, les antiarhythmiques, les antiangineux, les diurétiques, les antihypertensifs, les anticoagulants, les antithrombotiques, les fibrinolytiques, les hémostatiques, les agents hypolipidémiques, les agents antianémiques et neurotropéniques, les hypnotiques, les anxiolytiques, les antipsychotiques, les antidépresseurs, les antiémétiques, les anticonvulsivants, les stimulants du SNC, les analgésiques, les antipyrétiques, les agents antimigraineux, les agents antiinflammatoires non stéroïdiens, les agents antigoutte, les myorelaxants, les agents neuromusculaires, les stéroïdes, les agents hypoglycémiques, les agents hyperglycémiques, les agents de diagnostic, les antibiotiques, les antifongiques, les antimalariques, les antiviraux, les immunosuppresseurs, les agents nutritionnels, les vitamines, les électrolytes, les anorexigènes, les modérateurs de l'appétit, les bronchodilatateurs, les expectorants, les antitussifs, les mucolytes, les décongestionnants, les agents anti-glaucome, les agents contraceptifs oraux, les agents de diagnostic et néoplasiques.

11. Procédé selon la revendication 8, dans lequel les perles, les perles ou microcapsules enrobées d'une membrane présentent une taille de particules dans la gamme allant de 50 à 1 500 μm.

12. Procédé selon la revendication 9, dans lequel la membrane comprend une fonction choisie parmi une fonction masquant le goût, une fonction de protection entérique, une fonction de libération maîtrisée pour permettre la libération d'un principe actif provenant de la forme de dosage sur une durée prolongée et une fonction de libération progressive pour permettre la libération d'un principe actif sur le site ciblé le long du tractus gastro-intestinal.

13. Procédé selon l'une quelconque des revendications précédentes, dans lequel le noyau de comprimé comprend une matière polymère qui gonfle au contact d'un liquide aqueux, choisie parmi la carboxyméthylcellulose sodique réticulée, l'hydroxypropylcellulose réticulée, l'hydroxypropylcellulose à masse moléculaire élevée, le carboxyméthylamide, le copolymère méthacrylate de potassium-divinylbenzène, le poly(méthacrylate de méthyle), la polyvinylpyrrolidone réticulée et les poly(alcools vinyliques) à masse moléculaire élevée.

**14.** Procédé selon l'une quelconque des revendications précédentes, dans lequel le revêtement est rapidement hydrosoluble.

**15.** Procédé selon l'une quelconque des revendications précédentes, dans lequel le revêtement comprend une résine polymère choisie parmi les polyméthacrylates, la cellulose et ses dérivés, les éthers et esters cellulosiques et l'acétophtalate de cellulose.

FIG. 1

FIG. 2

FIG. 3

FIG. 4

FIG. 5